(19) Europäisches Patentamt
European Patent Office
Office européen des brevets

(11) **EP 2 512 332 B1**

(12) **EUROPEAN PATENT SPECIFICATION**

(45) Date of publication and mention
of the grant of the patent:
**17.10.2018 Bulletin 2018/42**

(21) Application number: **10795175.8**

(22) Date of filing: **14.12.2010**

(51) Int Cl.:
**A61B 5/0476** (2006.01)

(86) International application number:
**PCT/US2010/060170**

(87) International publication number:
**WO 2011/084394 (14.07.2011 Gazette 2011/28)**

(54) **DEVICE FOR POINT-OF-CARE NEURO-ASSESSMENT AND TREATMENT GUIDANCE**

VORRICHTUNG ZUR ANLEITUNG VON POINT-OF-CARE-NEUROUNTERSUCHUNG UND -BEHANDLUNG

DISPOSITIF POUR L'ÉVALUATION NEUROLOGIQUE D'INTERVENTION ET LE GUIDAGE DU TRAITEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2009 US 639357**

(43) Date of publication of application:
**24.10.2012 Bulletin 2012/43**

(73) Proprietors:
• **Brainscope Company, Inc.**
**Bethesda, MD 20814-4481 (US)**
• **New York University**
**New York City, NY 10012 (US)**

(72) Inventors:
• **PRICHEP, Leslie**
**Mamaroneck**
**NY 10543 (US)**

• **CAUSEVIC, Elvir**
**Denver, CO 80209 (US)**

(74) Representative: **Finnegan Europe LLP**
**1 London Bridge**
**London SE1 9BG (GB)**

(56) References cited:
**EP-A1- 1 275 340         WO-A1-91/09372**
**WO-A1-2010/088252    US-A- 4 188 956**
**US-A- 4 913 160           US-A1- 2005 165 323**
**US-A1- 2007 032 737    US-A1- 2007 100 251**
**US-A1- 2008 208 073    US-A1- 2009 082 690**
**US-A1- 2009 221 930    US-A1- 2009 263 034**
**US-A1- 2009 264 786**

**Description**

Technical Field

The present disclosure relates to the field of neurological assessment, and specifically, to a portable apparatus and method for performing neurological assessment on a patient at the point-of-care.

Background

The brain performs the most complex and essential processes in the human body. Surprisingly, contemporary health care lacks sophisticated tools to objectively assess brain function at the point-of-care. A patient's mental and neurological status is typically assessed by an interview and a subjective physical exam. Clinical laboratories currently have no capacity to assess brain function or pathology, contributing little more than identification of poisons, toxins, or drugs that may have externally impacted the central nervous system (CNS).

Brain imaging studies, such as computed tomography (CT) and magnetic resonance imaging (MRI), are widely used to visualize the structure of the brain. However, CT scan and MRI are anatomical tests and reveal very little information about brain function. For example, intoxication, concussion, active seizure, metabolic encephalopathy, infections, and numerous other conditions (e.g. diabetic coma) show no abnormality on CT scan. A classical stroke, or a traumatic brain injury (TBI), may not be immediately visualized by an imaging test even if there is a clear and noticeably abnormal brain function. Similarly, diffuse axonal injury (DAI), related to shearing of nerve fibers which is present in majority of concussive brain injury cases, can remain invisible on most routine structural images. If undetected at an early stage, swelling or edema from DAI can subsequently lead to coma and death.

Functional MRI (fMRI) is a recent improvement over MRI, which provides relative images of the concentration of oxygenated hemoglobin in various parts of the brain. While the concentration of oxygenated hemoglobin is a useful indication of the metabolic function of specific brain regions, it provides very limited information about the underlying electrochemical processes within the brain.

Further, CT and MRI/fMRI testing devices are not field-deployable due to their size, power requirements and cost. These assessment tools play an important role in selected cases, but they are not universally available, require experienced personnel to operate, and they do not provide critical information at the early stages of acute neurological conditions. Current technologies are unable to provide the immediate information critical to timely intervention, appropriate triage, or the formulation of an appropriate plan of care for acute brain trauma. Unfortunately, the brain has very limited capacity for repair, and thus time-sensitive triage and intervention is very important in treating brain injuries.

Currently, emergency room patients with altered mental status, acute neuropathy, or head trauma must undergo costly and time-consuming tests to determine an appropriate course of treatment. Unfortunately, in many cases, the clinical condition of patients continue to deteriorate as they wait for equipment to become available or for specialists to interpret tests. The problem that faces ER physicians is that their resources are limited to a subjective physical exam using a flashlight and a reflex hammer, and all of the physician's decisions concerning the administration of emergency treatment, additional consultation by a neurologist, or patient discharge, are based on the results of this simplistic exam. Often, ER patients are sent for imaging studies, yet many functional brain abnormalities, as discussed earlier, are not visible on a CT scan or MRI. Some abnormalities which eventually have anatomical and structural consequences often take time to become visible on an imaging test. This is true for many important conditions, such as ischemic stroke, concussion/traumatic brain injury (TBI), raised intracranial pressure, and others. This indicates the need for real-time, functional brain state assessment technology, which can be performed in the ER, or in an ambulatory setting, and can detect emergency neurological conditions hours ahead of the standard clinical assessment tools available today. Similarly, there is a need for a point-of-care assessment tool for detection of TBI in soldiers out in the battlefield, and also for detection of sports-related brain injury in athletes. Rapid, on-the-field assessments may help prevent repeat injuries and "second impact syndrome" in soldiers and athletes already suffering from a first traumatic brain impact.

All of the brain's activities, whether sensory, cognitive, emotional, autonomic, or motor function, is electrical in nature. Through a series of electrochemical reactions, mediated by molecules called neurotransmitters, electrical potentials are generated and transmitted throughout the brain, traveling continuously between and among the myriad of neurons. This activity establishes the basic electrical signatures of the electroencephalogram (EEG) and creates identifiable frequencies which have a basis in anatomic structure and function. Understanding these basic rhythms and their significance makes it possible to characterize the brain electrical signals as being within or beyond normal limits. At this basic level, the electrical signals serve as a signature for both normal and abnormal brain function. Just as an abnormal electrocardiogram (ECG) pattern is a strong indication of a particular heart pathology, an irregular brain wave pattern is a strong indication of a particular brain pathology.

Even though EEG-based neurometric technology is accepted today in neurodiagnostics, application in the clinical environment is notably limited. Some of the barriers limiting its adoption include: the cost of EEG equipment, the need for a skilled technician to administer the test, the time it takes to conduct the test, and the need for expert interpretation of the raw data. The instrument produces essentially raw waveforms which must be carefully interpreted by an expert. Data is collected and analyzed by an EEG technician, and is then presented to a neurologist for interpretation and clinical assessment. This makes the currently available EEG equipment unfeasible for neuro-triage applications in emergency rooms or at other point-of-care settings. More importantly, the current technology is not field-portable which makes it unfeasible for various field applications, e.g., at a battle field, or a sports event. Thus, there is an immediate need for a brain state assessment technology for providing rapid, point-of-care neurological triage and treatment guidance for patients with acute brain injury or disease, so as to prevent further brain damage and disability.

US 2007/0032737 discloses a neurological triage apparatus. An electrode set, which may comprise fewer than nineteen electrodes, is placed on a patient. The electrodes measure the electrical fields that are produced as a result of brain activity. Artifacts are removed from the signals acquired by the electrode set, and statistical signal features are extracted. The features are classified according to one or more diagnostic categories. The classification may be performed using discriminant analysis.

US 2008/0208073 discloses a portable device for detecting the presence and/or severity of a concussion in a subject. The device comprises a headset having a plurality of neurological signal-detecting electrodes and a handheld base unit. The base unit comprises a display that provides a color-coded indication of the presence and/or severity of a concussion. A red indication is displayed if the concussion is present and serious; an orange indication is displayed if a concussion is likely present and more tests are required to be performed on the subject; and a green indication is displayed if there is no concussion.

Summary of the Invention

The present disclosure addresses the need for point-of-care neuro-triage by providing a portable device for rapid, real-time evaluation of the brain electrical signals of a patient.

In accordance with the invention, there is provided an apparatus for performing neurological triage on a patient, as recited by claim 1.

A first aspect of the present disclosure not forming part of the claimed invention includes a method for performing neurological triage on a patient. The method comprises the steps of providing a patient sensor comprising at most eight disposable neurological electrodes and at least one ear phone, acquiring spontaneous brain electrical signal using the neurological electrodes, providing a handheld base unit comprising a signal processor, the base unit being operatively coupled to the patient sensor for processing the acquired spontaneous brain electrical signals, and further calculating an index indicating a statistical probability of the patient's brain electrical signals being normal or abnormal using discriminant classification analysis, and displaying the index on the handheld base unit.

Another aspect of the present invention comprises an apparatus for performing neurological triage on a patient. The apparatus comprises a patient sensor comprising at most eight disposable neurological electrodes and at least one ear phone, and a handheld base unit operatively connected to the patient sensor. The base unit further comprises a digital signal processor configured to perform automatic identification and removal of artifacts from acquired spontaneous brain electrical signals, discriminant-based classification using pre-selected subsets of quantitative signal features, and calculating an index capable of indicating a statistical probability of the patient's brain electrical signals being normal or abnormal, and indicate, when the index is greater than 10% or less than 90%, that the classification does not allow the presence or absence of an abnormality to be determined. Additionally, the base unit comprises a display panel to display the index.

It is to be understood that both the foregoing general description and the following detailed description are exemplary and explanatory only and are not restrictive of the invention, as claimed.

The accompanying drawings, which are incorporated in and constitute a part of this specification, illustrate embodiments of the invention and together with the description, serve to explain the principles of the various aspects of the invention.

Brief Description of Drawings

FIG. 1A shows an exemplary embodiment of a neuro-assessment apparatus for acquiring and processing brain electrical signals;

FIG. 1B shows the placement of electrodes on a subject's forehead, in accordance with the International 10/20 electrode placement system;

FIG. 2A illustrates a graphic data summary displayed on the screen of a neuro-assessment apparatus in accordance with an exemplary embodiment of the present disclosure;

FIG. 2B illustrates the display of detailed date regarding quantitative signal features on the screen of a neuro-assessment apparatus in accordance with an exemplary embodiment of the present disclosure;

FIG. 2C illustrates Z-score Normalized Maps displayed on the screen of a neuro-assessment apparatus in accordance with an exemplary embodiment of the present disclosure; and

FIG. 3 shows a flowchart diagramming the steps of performing neurological triage on a subject using a handheld device in accordance with an exemplary embodiment of the present disclosure.

Detailed Description

Reference will now be made in detail to embodiments consistent with the present invention, examples of which are illustrated in the accompanying drawings. Wherever possible, the same reference numbers will be used throughout the drawings to refer to the same or like parts.

In an exemplary embodiment, data corresponding to brain electrical activity is used to detect acute neurological injury or disease in patients. The brain electrical signals are measured and analyzed at the point-of-care using a portable neuro-triage device developed using Bx™ technology, so as to provide an immediate evaluation of the subject's neurological condition. In accordance with an exemplary embodiment of the Bx™ technology, a subject's brain electrical activity is recorded using a varying number of electrodes located at standardized positions on the scalp and forehead, and the subject's brain electrical signals are assessed with reference to one or more databases. For example, collected normative data, indicative of normal brain electrical activity, is used to establish quantitative features of brain electrical activity, which clearly distinguish brain signals produced in the presence and absence of acute neurological disorder. This normative dataset includes brain activity data of a control group of population. A normative population in the database comprises of individuals similar to a subject in one or more aspects, such as age, gender, etc. In one exemplary embodiment, a subject is compared to individuals in the database using a regression equation as a function of age. The collected normative database employed by the inventor has been shown to be independent of racial background and to have extremely high test-retest reliability, specificity (low false positive rate) and sensitivity (low false negative rate).

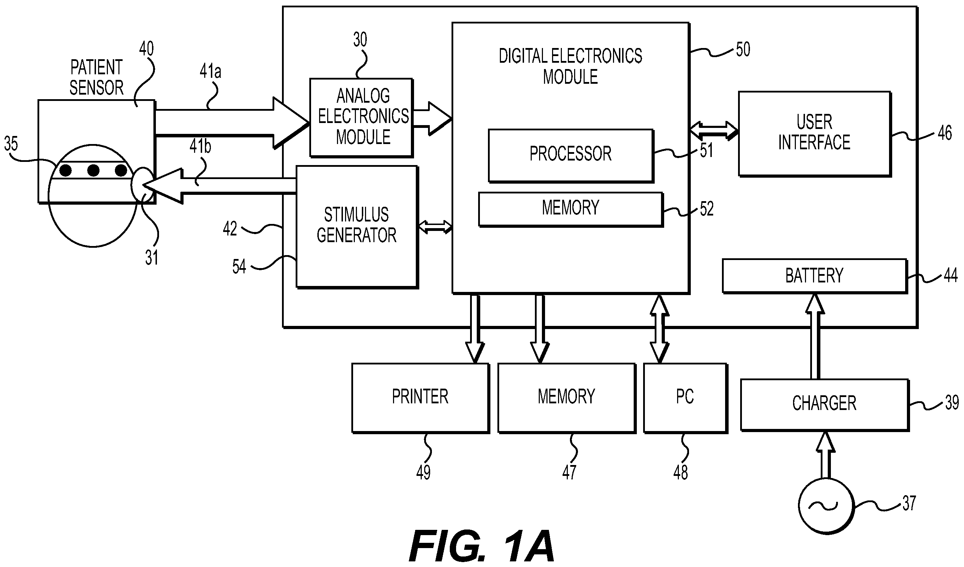

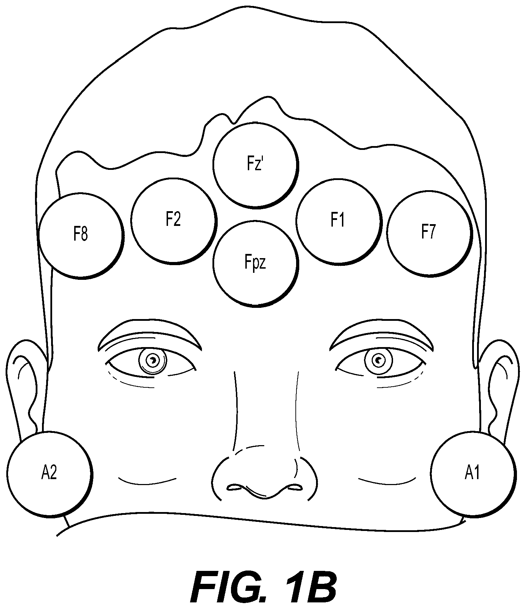

In accordance with embodiments consistent with the present disclosure, FIG. 1A shows a neuro-assessment apparatus for acquiring and processing brain electrical signals using Bx™ technology, and providing an evaluation of the subjects neurological condition. In an exemplary embodiment, the neuro-assessment apparatus is implemented as a portable device for point-of-care applications. This apparatus consists of a patient sensor 40 which may be coupled to a base unit 42, which can be handheld, as illustrated in FIG. 1A. Patient sensor 40 may include an electrode array 35 comprising at least one disposable neurological electrode to be attached to a patient's head to acquire brain electrical signals. The electrodes are configured for sensing both spontaneous brain activity as well as evoked potentials generated in response to applied audio stimuli. In one exemplary embodiment, the apparatus comprises of five (active) channels and three reference channels. The electrode array 35 consists of anterior (frontal) electrodes: F1, F2, F7, F8, AFz (also referred to as Fz') and Fpz (reference electrode) to be attached to a subject's forehead, and electrodes A1 and A2 to be placed on the front or back side of the ear lobes, or on the mastoids, in accordance with the International 10/20 electrode placement system (with the exception of AFz). The electrode placement is illustrated in FIG. 1B. The use of a limited number of electrodes enable rapid and repeatable placement of the electrodes on a subject, which in turn facilitates efficient, and more accurate, patient monitoring. Further, in one embodiment, the electrodes may be positioned on a low-cost, disposable platform, which can serve as a "one-size-fits-all" sensor. For example, electrodes 35 may be positioned on a head gear that is configured for easy and/or rapid placement on a patient. Other electrode configurations may be utilized as and when required, as would be understood by those of ordinary skill in the art.

In an exemplary embodiment, the neuro-assessment device utilizes the advantages of auditory evoked potential (AEP) signals to map specific auditory, neurological and psychiatric dysfunctions. In such an embodiment, the patient sensor 40 includes reusable earphone 31 to provide auditory stimuli clicks in either ear. In some embodiments, the auditory evoked potential signal used is auditory brainstem response (ABR). In such embodiments, the auditory stimuli may be delivered at 100 dB Peak-to-Peak Equivalent Sound Pressure Level and at a frequency (rate) of 27 Hz (27 clicks per second) to evoke electrical signals from the brainstem in response to the applied auditory stimuli. In another embodiment, patient sensor 40 may include an additional ear phone to deliver white noise in the other ear.

The patient sensor 40 also includes two reusable patient interface cables which are designed to plug into the base unit 42 and provide direct communication between the patient sensor 40 and the base unit 42. The first cable is an electrical signal cable 41a, which is equipped with standard snap connectors to attach to the disposable electrodes placed on the patient's scalp. The second cable is the AEP stimulus cable 41b which provides connection to the earphone 31 for auditory stimulus delivery. Other auditory stimuli may also be used, to evoke mid-latency (20-80 milliseconds) or late auditory responses (>80 milliseconds), including the P300.

The base unit 42 primarily includes an analog electronics module 30, a digital electronics module 50, user interface 46, stimulus generator 54 and battery 44 as illustrated in FIG. 1A. The analog electronics module receives signals from one or more of the neurological electrodes operatively connected through the electrical cable 41a. The analog module is configured to amplify, filter, and preprocess the analog waveforms acquired from the electrode channels. The analog module may comprise signal amplifier channels including at least one preamplifier, at least one differential amplifier, at least one common mode detector, and at least one gain stage with filter. The analog amplifier channels correspond to the number of electrode channels. In an embodiment consistent with the present disclosure, there are 8 analog amplifier channels corresponding to 8 electrode channels (5 active, 3 reference channels). The analog module 30 may further include a multiplexer (MUX), which combines many analog input signals and outputs that into a single channel, and an analog-to-digital converter (ADC) to digitized the received analog signal. Digital electronics module 50 can then process the digitized data acquired through analog module 30 and can perform analysis of data to aid in interpretation of data pertaining to brain electrical activity. Further, as shown in FIG. 1A, the digital electronics module 50 may be operatively connected with a number of additional device components.

In an exemplary embodiment, the analog electronics module 30 is further configured to check an impedance by feeding a signal back into each electrode. Checking an impedance may function to characterize the effectiveness of connection of a surface electrode to a subject. This would enable an user to test the applied electrodes at a patient site before signal acquisition is started, and also monitor the electrode impedance continuously in real-time throughout the test. In an exemplary embodiment, the impedance of the applied electrodes are measured periodically and the impedance values are displayed on the user interface 46 using a color-coded electrode visual display, which allows the user to monitor the electrode impedances before and during a test session. If an impedance value is found to be unacceptable at the time of the measurement, a warning indication may be displayed on the screen instructing the user to check the electrode connection.

The digital electronics module 50 comprises a digital signal processor (DSP) 51 for processing the data corresponding to the acquired brain electrical signals, and a memory 52 which stores the instructions for processing the data, such as a DSP algorithm. The processor 51 is configured to perform the following tasks-

a) Automatic identification and removal of several types of signal artifacts from the acquired spontaneous brain electrical signal data;
b) Extraction of linear and non-linear signal features; and
c) Linear and non-linear discriminant analysis-based classification using pre-selected subsets of age-normalized features (z-scores).

In some embodiments, the processor 51 is further configured to process the acquired auditory evoked potential signals. For example, in some embodiments, processor 51 is configured for reconstruction of acquired ABR waveforms, removal of epochs containing artifacts, filtering, synchronized averaging and computation of Fsp, which is a measure of reconstructed signal quality. Similarly, in some embodiments, processor 51 is configured to process other auditory evoked potentials.

The processor 51 is configured to implement the DSP algorithm to identify data that is contaminated by non brain-generated artifacts, such as eye movements, electromyographic activity (EMG) produced by muscle tension, spike (impulse), external noise, etc., as well as unusual electrical activity of the brain not part of the estimation of stationary background state. Artifact identification is performed using as input the signals from the five active leads Fp1, Fp2, F7, F8, AFz referenced to linked ears (A1+A2)/2, and sampled at 100 Hz. In one embodiment, incoming data epochs of 2.56 seconds (256 samples per epoch) are split into 8 basic data units (sub-epochs) of length 320 ms (32 data points per sub-epoch). Artifact identification is done on a per-sub-epoch basis and guard bands are implemented around identified artifact segments of each type. Artifact-free epochs are then constructed from at most two continuous data segments, with each data segment being no shorter than 960 ms (which corresponds to the time span of 3 contiguous sub-epochs). The resulting artifact-free data is then processed to extract signal features and classify the extracted features to provide a triage result.

In another embodiment, signal denoising is performed using a signal processing method described in commonly-assigned U.S. Patent Application No. 2009/0263034. In one embodiment consistent with the present disclosure, the artifact identification and rejection algorithm follows the following steps:

a. Transforming the signal into a plurality of signal components;
b. Computing fractal dimension of the components;
c. Identifying noise components based on their fractal dimension;
d. Automatically attenuating the identified noise components;
e. Reconstructing a denoised signal using inverse transform.

The input analog brain electrical signal is at first digitized and then deconstructed into its constitutive coefficients using a linear or non-linear signal transformation method, such as Fast Fourier Transform, Independent Component Analysis (ICA)-based transform, wavelet transform, wavelet packet transform etc. The fractal dimensions of the coefficients are then calculated in the transform domain, and the coefficients that have a fractal dimension higher than a preset threshold value are attenuated. The intact and re-scaled coefficients are then remixed using an inverse signal transform to generate a denoised signal, which is further processed to extract signal features and classify the extracted features.

Processor 51 is configured to execute instructions contained in memory 52 to perform an algorithm for quantitative feature extraction from processed signals. In one embodiment, the algorithm extracts various quantitative features from the brain wave frequency bands: Delta (1.5-3.5 Hz), Theta (3.5-7.5 Hz), Alpha (7.5-12.5 Hz), Alpha1 (7.5-10 Hz), Alpha2 (10-12.5 Hz), Beta (12.5-25 Hz), Beta2 (25-35 Hz), Gamma (35-50 Hz), and high frequency EEG (>50 Hz). In some embodiments, the features computed are: absolute power, relative power, mean frequency, coherence, symmetry, fractal dimension, wavelet features, and several statistical harmonics variables. The feature extraction algorithm takes as input a number of "artifact-free" or "denoised" epochs having a temporal length of 2.56 seconds, which corresponds to 256 samples for data sampled at 100 Hz. In an exemplary embodiment, processor 51 is configured to perform a linear feature extraction algorithm based on Fast Fourier Transform (FFT). In another embodiment, processor 51 is configured to perform a non-linear feature extraction algorithm based on wavelet transforms, such as Discrete Wavelet Transform (DWT), Complex Wavelet Transforms (CWT), Biorthogonal Discrete Wavelet Transform (BDWT), Wavelet Packet Decomposition, etc. A full set of monopolar and bipolar features are calculated and then transformed for Gaussianity. Once a Gaussian distribution has been demonstrated and age regression applied, statistical Z transformation is performed to produce Z-scores. The Z-transform is used to describe the deviations from age expected normal values:

Z= Probability that subject value lies within the normal range

$$Z = \frac{\text{Subject Value - Norm for Age}}{\text{Standard Deviation for Age}}$$

The Z-scores are calculated for each feature and for each electrode using a database of response signals from a large population of subjects believed to be normal, or to have other pre-diagnosed conditions. In particular, each extracted feature is converted to a Z-transform score, which characterizes the probability that the extracted feature observed in the subject will conform to a normal value.

Processor 51 is further configured to perform a discriminant-based classification algorithm wherein the extracted features, or the Z-scores, are classified. In one embodiment, the classification is performed using Linear Discriminant Analysis (LDA), which optimally combines the features (Z-scores) into a discriminant score that possesses the maximum discriminating power. Linear Discriminant Analysis is a two category classifier, such as a classification between normal and abnormal, which assigns for each given subject a discriminant score between 1 and 100. For example, the discriminant scores, $S_N$ and $S_{AB}$ corresponding to classes "normal" and "abnormal", are computed for any subject with the following Fisher LDA formulas:

$$S_N = 100 \cdot G(1)/(G(1) + G(2)), \quad S_{AB} = 100 \cdot G(2)/(G(1)+G(2))$$

$$G(1) = \exp(Z \cdot W_N + C_N), \quad G(2) = \exp(Z \cdot W_{AB} + C_{AB})$$

where Z denote the set of age-regressed Z-transformed features (discriminants) computed for any subject. $W_N$ and $W_{AB}$ denote two weight vectors that are derived from a stored reference database, and $C_N$ and $C_{AB}$ are two constants which are commonly called bias or threshold weights. The weights for the different monopolar and/or bipolar univariate and multivariate features are pre-selected using a training routine such that they result in the 'best' separation between the classes. The weights may be estimated from a stored population reference database, such as a database comprising of population normative data indicative of brain electrical activity of a first plurality of individuals having normal brain state, or population reference data indicative of brain electrical activity of a second plurality of individuals having an abnormal brain state. Similarly, the weights may be selected from a database of the subjects own brain electrical activity data generated in the absence or presence of an abnormal brain state. In some embodiments, the classification task may be performed using one or more discriminant functions, and in such a case, the discriminant outputs may be combined using a majority voting rule.

In an exemplary embodiment, processor 51 is configured to calculate an index indicating a statistical probability of the patient's brain electrical signals being normal or abnormal, known as the "Probability of Normal" index. In certain embodiments, the "Probability of Normal" index is calculated from the discriminant score using Receiver Operating Characteristics (ROC) curves and Classification Accuracy Curves (CAC), as described in U.S. Application No. 2010/0191139. Using this method, the "Probability of Normal" for any normal/abnormal classification can be derived, which is an integer in the range 0-100. Additionally, in some embodiments, processor 51 is configured to identify one or more features making the largest contribution to the "Probability of Normal" classification statement (whenever the index is smaller than 10% or larger than 90%).

In addition to the acquisition and processing of spontaneous brain electrical signals, the device collects auditory evoked potential response data. For example, in some embodiments, the device collects auditory brainstem response (ABR) data and displays the averaged ABR waveforms. For each of the two modalities ("Left ABR," and "Right ABR"), raw data is collected for approximately 2.5 minutes (corresponding to 4096 raw ABR epochs). The ABR waveform is constructed and displayed for lead AFz only, using contra-lateral referencing, which means that for the "Left ABR" modality where the acoustic stimulus is in the left ear, the device computes and displays the ABR waveform for the signal AFz - A2 (by synchronized averaging of artifact-free epochs). Similarly, for the Right ABR modality, the waveform for AFz - A1 is computed and displayed. At the end of the ABR data acquisition process, the device computes and displays the Fsp next to the waveform. For the computation of the ABR waveform the following processing steps are performed: bandpass-filtering of raw ABR epochs, rejection of artifacted ("over-range") epochs, followed by Bayesian averaging of the remaining artifact-free epochs. Optionally, adaptive filtering may be performed for ABR waveform reconstruction.

The memory 52 may further contain interactive instructions for using and operating the device to be displayed on a screen of the user interface 46. The instructions may comprise an interactive feature-rich presentation including a multimedia recording providing audio/video instructions for operating the device, or alternatively simple text, displayed on the screen, illustrating step-by-step instructions for operating and using the device. The inclusion of interactive instructions with the device eliminates the need for extensive training for use, allowing for deployment and use by persons other than medical professionals. The memory 52 may also contain a database, which includes the collected normative data and reference data used for feature classification. In an exemplary embodiment, the database may be accessed from a remote storage device via a wireless or a wired connection. Similarly, data collected from the subject by the neuro-triage apparatus may be recorded in the database for future reference.

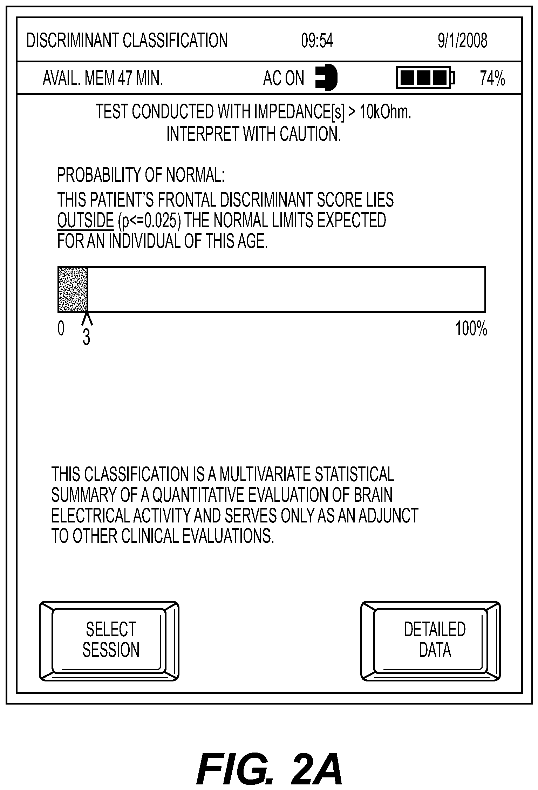

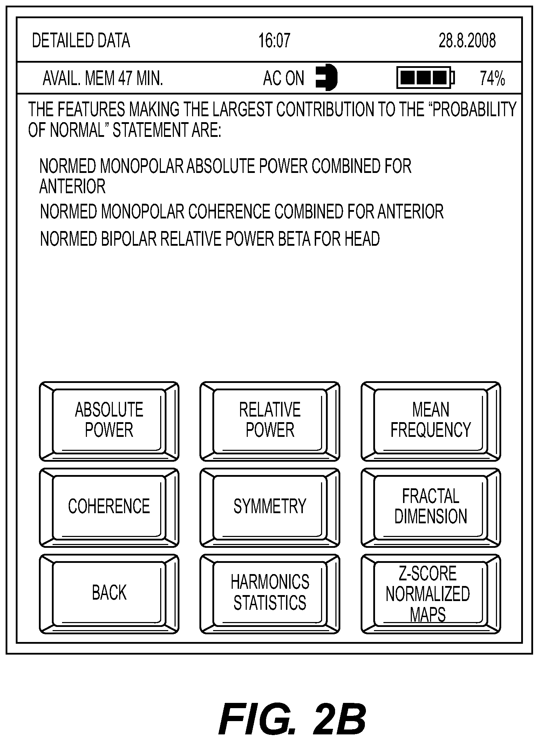

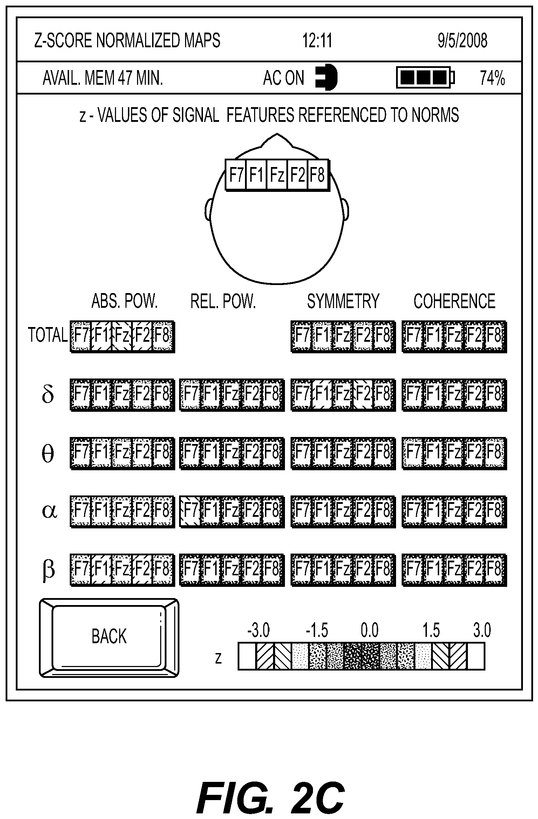

The neuro-triage device can be a standalone system or can operate in conjunction with a mobile or stationary device to facilitate display or storage of data, and to signal healthcare personnel when therapeutic action is needed, thereby facilitating early recognition of emergency conditions. Mobile devices can include, but are not limited to, handheld devices and wireless devices distant from, and in communication with, the neuro-triage device. Stationary devices can include, but are not limited to, desktop computers, printers and other peripherals that display or store the results of the neurological evaluation. In an exemplary embodiment, the neuro-triage device stores each patient file, which includes a summary of the session and test results, on a removable memory card 47, such as compact flash (CF) card. The user can then use the memory card 47 to transfer patient information and procedural data to a computer, or to produce a printout of the data and session summary. In another embodiment, results from the processor 51 are transferred directly to an external mobile or stationary device to facilitate display or storage of data. For example, the results from the processor 51 may be displayed or stored on a PC 48 connected to the base unit 42 using a PC interface, such as an USB port, IRDA port, BLUETOOTH® or other wireless link. In yet another embodiment, the results can be transmitted wirelessly or via a cable to a printer 49 that prints the results to be used by attending medical personnel. In an embodiment consistent with the present disclosure, user interface 46 may be configured to communicate patient information and/or procedural data to an attending medical personnel, such as an ER physician, a triage nurse, or an emergency response technician. Information that is conveyed through user interface 46 can include a variety of different data types, including, but not limited to, diagnostic results, intermediate analysis results, usage settings, etc. In an exemplary embodiment, the user interface 46 displays the brain electrical signal graphs drawn in real-time for the five active and the three reference channels, along with the Right ABR waveform and Left ABR waveform graphs, Fsp value and the actual number of clean epochs used for the computation of the ABR waveforms. Additionally, as shown in FIG. 2A, the screen provides a graphic data summary, which includes a Discriminant Classification screen showing a horizontal index bar with numerical indications of "Probability of Normal" (PoN). The display of the index is accompanied by a message which states the statistical interpretation of the index. In some exemplary embodiments, the index is an integer in the range 0-100 and is graphically represented by a black bar in a 0-100 scale. For patients whose frontal discriminant score is defined by a PoN greater than 10% and lesser that 90%, the device displays a Data Classification message notifying the user that the discriminant score does not allow a confident determination of the presence of abnormality. The user interface 46 further displays a Detailed Data screen, as illustrated in FIG. 2B, which provides access to detailed data about the features that made the largest contribution to the abnormal classification. From this screen, the user would be able to access tabular screens showing values of the quantitative features and the Z-scores for each feature extracted from the artifact-free data epochs. The Detailed Data screen allows the user to select tables for Absolute Power, Relative Power, Mean Frequency, Coherence, Symmetry, Fractal Dimension and Harmonics Statistics. The frequencies included in this tables are Delta (1.5-3.5 Hz), Theta (3.5-7.5 Hz), Alpha (7.5-12.5 Hz), Beta (12.5-35 Hz), Gamma (35-50 Hz), and S (1.5-25 Hz). From the Detailed Data screen, the user will also be able to navigate to the Z-scores Normalized Maps screen, which is shown in FIG. 2C. This screen gives the user an option to view a graphical representation of a pre-selected subset of Z-scores on a "frontal head map" where Z-scores are color-coded to show deviation from normal.

Additionally, in some embodiments, the user interface 46 gives the user the option to view and statistically compare multiple sessions of an individual for the purpose of treatment evaluation or progression of disorder.

In another exemplary embodiment, user interface 46 may receive and display usage setting information, such as the name, age and/or other statistics pertaining to the patient. The user interface 46 comprises a touchscreen interface for entering the user input. A virtual keypad may be provided on the touchscreen interface for input of patient record fields. Additionally, as shown in FIG. 2A, the battery charge status may be indicated continuously on the display, along with the available memory status of the CF card, and the electrode impedance values. Further, the neuro-assessment device can transmit data to another mobile or stationary device to facilitate more complex data processing or analysis. For example, the neuro-assessment device, operating in conjunction with PC 48, can send data to be further processed by the computer. In another embodiment consistent with the Bx™ technology, the processor 50 transmits a raw, unprocessed signal acquired from a subject to PC 48 for analyzing the recorded data and outputting the results. The unprocessed brain electrical signals recorded from a subject may also be stored in a remote database for future reference and/or additional signal processing.

In an embodiment consistent with the present disclosure, the base unit 42 includes a stimulus generator 54, which is operatively coupled to the processor 51, for applying auditory stimuli to the subject to elicit ABR waveforms. The stimulus generator 54 interfaces with earphone 31 positioned in proximity to the patient's ear to deliver auditory stimuli that can generate evoked potentials. The processor 51 then removes artifacts from the received evoked potential signala and displays the artifact-free waveforms, as described earlier in this paper. Additionally, base unit 42 contains an internal rechargeable battery 44 that can be charged during or in between uses by battery charger 39 connected to an AC outlet 37.

The neuro-assessment apparatus, developed in accordance with the Bx™ technology, is designed for near-patient testing in emergency rooms, ambulatory setting, and other field applications. The neuro-assessment device is intended to be used in conjunction with CT scan, MRI or other imaging studies to provide complementary or corroborative information about a patient's neurological condition. The key objective of point-of-care neuro-assessment is to provide fast results indicating the severity of a patient's neurological condition, so that appropriate treatment can be quickly provided, leading to an improved overall clinical outcome. For example, the neuro-assessment device may be used by an EMT, ER nurse, or any other medical professional during an initial patient processing in the ER or ambulatory setting, which will assist in identifying the patients with emergency neurological conditions. It will also help ER physicians in corroborating an immediate course of action, prioritizing patients for imaging, or determining if immediate referral to a neurologist or neurosurgeon is required. This in turn will also enable ER personnel to optimize the utilization of resources (e.g., physicians' time, use of imaging tests, neuro consults, etc.) in order to provide safe and immediate care to all patients.

In addition, the neuro-assessment device is designed to be field-portable, that is, it can be used in locations far removed from a full-service clinic-for example, in remote battlefield situations distant from military healthcare systems, during sporting events for indentifying if an injured athlete should be transported for emergency treatment, at a scene of mass casualty in order to identify patients who need critical attention and immediate transport to the hospital, or at any other remote location where there is limited access to well-trained medical technicians.

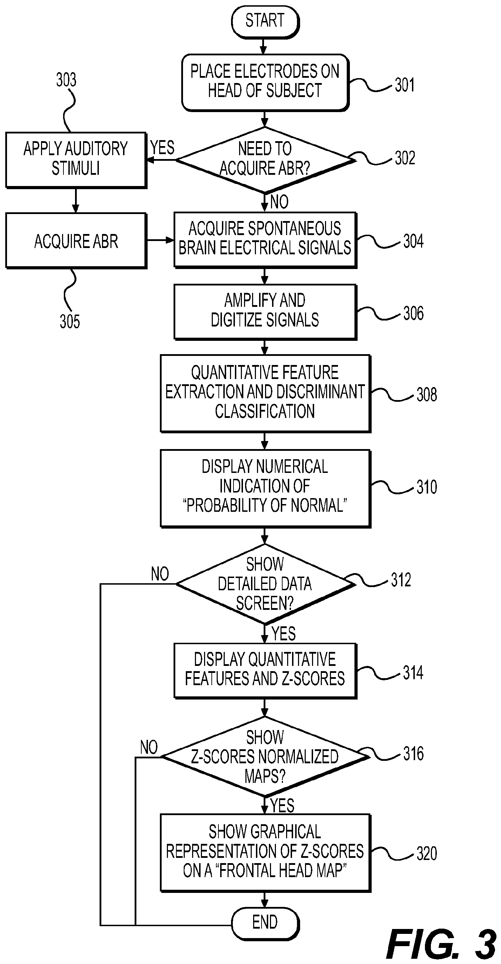

FIG. 3 shows a flowchart diagramming the steps of performing neurological triage on a subject using a handheld device, in accordance with an embodiment of the present invention, and will be described in conjunction with FIG. 1 to illustrate the method. The electrodes 35 are first placed on the head of the patient (step 301). The handheld base 42 unit is then powered on using power supplied by the battery 44. The processor 51 executes instructions stored in the memory 52 to display instructions for operating the device. An user can use the user interface 46 to enter a command to start signal acquisition. If the user determines that auditory evoked potentials (for example, ABR signals) may also have to be recorded (step 302), he may initiate stimulus generator 54 and apply auditory stimuli to elicit evoked potential responses (step 303). Brain electrical signals, which may include the spontaneous brain electrical signals and the evoked potential waveforms, are acquired using electrodes 35 (step 304 and 305), and the signals are then amplified and digitized in the handheld base unit 42 (step 306). The processor 51 is configured for processing the signal (i.e. feature extraction and discriminant-based classification) (step 308) using instructions stored in memory 52. The user interface 46 then displays a horizontal index bar with numerical indications of "Probability of Normal" (PoN) (step 310), which specifies the statistical value of probability of a patient's brain electrical signals being normal or abnormal. If an user wants to view the detailed data showing quantitative features and Z-scores (step 312), he may navigate to the Detailed Data screen (step 314) which shows the quantitative features that made the most contribution towards the determination of PoN. From this screen the user will also be able to access table sets showing values of quantitative features and Z-scores. The user will also be given an option to see a Z-score Normalized Maps (step 316), which shows a graphical representation of a pre-selected subset of Z-scores on a "frontal head map". Following the display of the result indicating the probability of brain activity being normal or abnormal, and optionally the detailed data showing the values of quantitative features and Z-scores Normalized Maps, the user may terminate the test and incorporate the result with data from other clinical tests, or he may repeat the test session to perform additional evaluation.

Embodiments consistent with the present disclosure, using advanced signal processing algorithms and stored data of the brain activity of thousands of subjects having different neurological indications, may provide a rapid and accurate assessment of the brain state of a subject. The advanced signal processing algorithms may be executed by a processor capable of integration in a portable handheld device. The portable handheld device used with a reduced electrode set allows for a rapid, on-site neurological triage, and determining an appropriate course of treatment at the early stage of an injury or other acute neurological disorder requiring immediate medical attention.

Other embodiments of the invention will be apparent to those skilled in the art from consideration of the specification and practice of the invention disclosed herein. It is intended that the specification and examples be considered as exemplary only, with a true scope of the invention being indicated by the following claims.

**Claims**

1.  An apparatus for performing neurological triage on a patient, comprising:

    a patient sensor (40) comprising at most eight disposable neurological electrodes (35) and at least one ear phone (31);
    a handheld base unit (42) operatively connected to the patient sensor (40), wherein the base unit (42) comprises a digital signal processor (51) configured to

        perform automatic identification and removal of artifacts from brain electrical signals acquired from the patient by the patient sensor, and
        perform discriminant-based classification using pre-selected subsets of quantitative signal features to classify the acquired brain signals as being normal or abnormal,

    calculate an index based on the discriminant-based classification, the index capable of indicating a statistical probability of the patient's brain electrical signals being normal or abnormal, wherein the base unit (42) further comprises a display panel configured to display the index, **characterised in that**:

        the digital signal processor (51) is further configured to
        indicate, when the index is greater than 10% or less than 90%, that the classification does not allow the presence or absence of an abnormality to be determined.

2.  The apparatus of claim 1, wherein the base unit (42) further comprises a stimulus generator (54).

3.  The apparatus of claim 1, wherein the digital signal processor (51) is further configured to process auditory evoked potential signals.

4.  The apparatus of claim 3, wherein the digital signal processor is further configured to display the auditory evoked potential signal waveform on the display panel.

5.  The apparatus of claim 1, wherein the display panel is further configured to display Z-transform scores of one or more quantitative features.

6.  The apparatus of claim 1, wherein interactive instructions for using and operating the device are stored in a memory (52) in the base unit (42); and wherein the interactive instructions are displayed on the display panel.

7.  The apparatus of claim 1, wherein probabilistic values of a set of quantitative features derived from the acquired brain electrical signals are displayed in the form of Z-transform scores on the display panel.

8.  The apparatus of claim 7, where the Z-transform scores are illustrated graphically in the form of a frontal head map displayed on the display panel of the handheld base unit (42).

9.  The apparatus of claim 1, wherein impedance values of the neurological electrodes are displayed on the handheld base unit (42) using a color-coded electrode visual display.

10. The apparatus of claim 1, wherein multiple sessions of the patient are graphically displayed on the display panel for comparison between evaluations.

11. The apparatus of claim 1, wherein the display panel is further configured to display tables of probabilistic values of a set of quantitative features derived from the acquired spontaneous brain electrical signals.

12. The apparatus of claim 1, further configured to:

provide auditory stimuli to the patient using the at least one ear phone (31);
acquire auditory evoked potential signals using the neurological electrodes (35); and
process the acquired auditory evoked potential signals using the handheld base unit (42).

13. The apparatus of claim 12, further configured to display the auditory evoked potential signal waveforms and a measure of the signal-to-noise ratio associated with the evoked potential signals.

14. The apparatus of claim 1, further configured to display the acquired brain electrical signals in real-time.

15. The apparatus of claim 1, wherein the digital signal processor (51) is configured to calculate the index by the steps of:

performing analog-to-digital conversion of the signals;
automatically identifying and removing artifacts from the signals; and
extracting quantitative features and computing Z-transform scores.

16. The apparatus of claim 15, wherein the computation of Z-transform scores and the discriminant classification is performed using a stored population database comprising brain electrical activity data from a plurality of individuals in the presence or absence of different types of acute neurological conditions.

17. The apparatus of claim 1, further configured to measure periodically the impedance of each electrode.


**Patentansprüche**

1. Vorrichtung zur Durchführung einer neurologischen Triage an einem Patienten, umfassend:

einen Patientensensor (40), umfassend höchstens acht verfügbare neurologische Einmal-Elektroden (35) und mindestens einen Kopfhörer 31);
eine tragbare Basiseinheit (42), die operativ mit dem Patientensensor (40) verbunden ist, wobei die Basiseinheit (42) einen Digitalsignalprozessor (51) umfasst, der konfiguriert ist, um eine automatische Identifikation und Entfernung von Artefakten aus elektrischen Hirnsignalen, die über den Patientensensor vom Patienten erworben wurden, durchzuführen und eine diskriminantenbasierte Klassifizierung mithilfe vorausgewählter Untergruppen quantitativer Signalmerkmale durchzuführen, um die erworbenen Hirnsignale als normal oder anormal zu klassifizieren, einen Index auf der Grundlage der diskriminantenbasierten Klassifizierung zu berechnen, wobei der Index in der Lage ist, eine statistische Wahrscheinlichkeit anzuzeigen, dass die elektrischen Hirnsignale des Patienten normal oder anormal sind, wobei die Basiseinheit (42) ferner ein Anzeigefeld umfasst, das konfiguriert ist, um den Index anzuzeigen, **dadurch gekennzeichnet, dass**:
der Digitalsignalprozessor (51) ferner konfiguriert ist, um anzuzeigen, wenn der Index größer als 10 % oder kleiner als 90 % ist, dass die Klassifizierung die Bestimmung eines Vorhandenseins oder einer Abwesenheit einer Anormalität nicht zulässt.

2. Vorrichtung nach Anspruch 1, wobei die Basiseinheit (42) ferner einen Stimulusgenerator (54) umfasst.

3. Vorrichtung nach Anspruch 1, wobei der Digitalsignalprozessor (51) ferner konfiguriert ist, um auditorisch hervorgerufene Spannungssignale zu verarbeiten.

4. Vorrichtung nach Anspruch 3, wobei der Digitalsignalprozessor ferner konfiguriert ist, um die auditorisch erzeugte Spannungssignalwellenform auf dem Anzeigefeld anzuzeigen.

5. Vorrichtung nach Anspruch 1, wobei das Anzeigefeld ferner konfiguriert ist, um Z-Transformationswerte eines oder mehrerer quantitativer Merkmale anzuzeigen.

6. Vorrichtung nach Anspruch 1, wobei interaktive Anweisungen zur Verwendung und Bedienung der Vorrichtung in einem Speicher (52) der Basiseinheit (42) gespeichert sind; und wobei die interaktiven Anweisungen auf dem Anzeigefeld angezeigt werden.

7. Vorrichtung nach Anspruch 1, wobei Wahrscheinlichkeitswerte einer Gruppe der quantitativen Merkmale aus den erworbenen elektrischen Hirnsignalen in Form von Z-Transformationswerten auf dem Anzeigefeld angezeigt werden.

8. Vorrichtung nach Anspruch 7, wobei die Z-Transformationswerte graphisch in Form einer auf dem Anzeigefeld der tragbaren Basiseinheit (42) angezeigten Vorderkopfabbildung illustriert werden.

9. Vorrichtung nach Anspruch 1, wobei Impedanzwerte der neurologischen Elektroden auf der tragbaren Basiseinheit (42) mithilfe einer farbkodierten visuellen Elektrodenanzeige angezeigt werden.

10. Vorrichtung nach Anspruch 1, wobei mehrere Sitzungen des Patienten zum Vergleich zwischen Auswertungen auf dem Anzeigefeld graphisch dargestellt werden.

11. Vorrichtung nach Anspruch 1, wobei das Anzeigefeld ferner konfiguriert ist, um Tabellen mit Wahrscheinlichkeitswerten einer Gruppe quantitativer Merkmale anzuzeigen, die aus den erworbenen spontanen elektrischen Hirnsignalen abgeleitet sind.

12. Vorrichtung nach Anspruch 1, ferner konfiguriert, um:
mithilfe des mindestens einen Kopfhörers (31) dem Patienten auditorische Stimuli bereitzustellen; auditorisch hervorgerufene Spannungssignale mithilfe der neurologischen Elektroden (35) zu erwerben und die erworbenen auditorisch hervorgerufenen Spannungssignale mithilfe der tragbaren Basiseinheit (42) zu verarbeiten.

13. Vorrichtung nach Anspruch 12, ferner konfiguriert, um die auditorisch hervorgerufenen Spannungssignalwellenformen und eine Messung des mit den hervorgerufenen Spannungssignalen verbundenen Signal-Rausch-Verhältnisses anzuzeigen.

14. Vorrichtung nach Anspruch 1, ferner konfiguriert, um die erworbenen elektrischen Hirnsignale in Echtzeit anzuzeigen.

15. Vorrichtung nach Anspruch 1, wobei der Digitalsignalprozessor (51) konfiguriert ist, um den Index durch die folgenden Schritte zu berechnen:

Durchführen einer Analog-zu-Digital-Umwandlung der Signale;
automatisches Identifizieren und Entfernen von Artefakten aus den Signalen; und
Extrahieren quantitativer Merkmale und Berechnen von Z-Transformationswerten.

16. Vorrichtung nach Anspruch 15, wobei die Berechnung der Z-Transformationswerte und der Diskriminanten-Klassifizierung mithilfe einer gespeicherten Populationsdatenbank durchgeführt wird, die elektrische Hirnaktivitätsdaten aus einer Vielzahl von Individuen in Anwesenheit oder Abwesenheit verschiedener Arten akuter neurologischer Zustände umfasst.

17. Vorrichtung nach Anspruch 1, ferner konfiguriert, um regelmäßig die Impedanz jeder Elektrode zu messen.

**Revendications**

1. Appareil permettant d'effectuer un triage neurologique sur un patient, comprenant :

un capteur de patient (40) comprenant au maximum huit électrodes neurologiques jetables (35) et au moins un écouteur (31) ;
une unité de base portative (42) reliée fonctionnellement au capteur de patient (40), ladite unité de base (42) comprenant un processeur de signaux numériques (51) conçu pour effectuer une identification automatique et la suppression des artefacts dus aux signaux électriques cérébraux acquis à partir du patient par le capteur de patient, et
effectuer une classification basée sur un discriminant à l'aide de sous-ensembles présélectionnés de caractéristiques de signaux quantitatives pour classer les signaux cérébraux acquis comme étant normaux ou anormaux,

calculer un indice sur la base de la classification basée sur un discriminant, l'indice pouvant indiquer une probabilité statistique des signaux électriques cérébraux du patient qui sont normaux ou anormaux, ladite unité de base (42) comprenant en outre un panneau d'affichage conçu pour afficher l'indice, **caractérisé en ce que** :
le processeur de signaux numériques (51) est en outre conçu pour indiquer, lorsque l'indice est supérieur à 10 % ou inférieur à 90 %, que la classification ne permet pas la détermination de la présence ou de l'absence d'une anomalie.

2. Appareil selon la revendication 1, ladite unité de base (42) comprenant en outre un générateur de stimulus (54).

3. Appareil selon la revendication 1, ledit processeur de signaux numériques (51) étant en outre conçu pour traiter des signaux de potentiels évoqués auditifs.

4. Appareil selon la revendication 3, ledit processeur de signaux numériques étant en outre conçu pour afficher la forme d'onde de signaux de potentiels évoqués auditifs sur le panneau d'affichage.

5. Appareil selon la revendication 1, ledit panneau d'affichage étant en outre conçu pour afficher des scores de transformée en Z d'une ou plusieurs caractéristiques quantitatives.

6. Appareil selon la revendication 1, des instructions interactives pour utiliser et faire fonctionner le dispositif étant stockées dans une mémoire (52) dans l'unité de base (42) ; et lesdites instructions interactives étant affichées sur le panneau d'affichage.

7. Appareil selon la revendication 1, les valeurs probabilistes d'un ensemble des caractéristiques quantitatives dérivées des signaux électriques cérébraux acquis étant affichées sous la forme de scores de transformée en Z sur le panneau d'affichage.

8. Appareil selon la revendication 7, les scores de transformée en Z étant illustrés graphiquement sous la forme d'une carte de la tête frontale affichée sur le panneau d'affichage de l'unité de base portative (42).

9. Appareil selon la revendication 1, les valeurs d'impédance des électrodes neurologiques étant affichées sur l'unité de base portative (42) à l'aide d'un affichage visuel des électrodes par codage couleur.

10. Appareil selon la revendication 1, de multiples sessions du patient étant affichées graphiquement sur le panneau d'affichage pour la comparaison entre les évaluations.

11. Appareil selon la revendication 1, ledit écran d'affichage étant en outre conçu pour afficher des tableaux de valeurs probabilistes d'un ensemble de caractéristiques quantitatives dérivées des signaux électriques cérébraux spontanés acquis.

12. Appareil selon la revendication 1, conçu en outre pour :

fournir des stimuli auditifs au patient à l'aide dudit au moins un écouteur (31) ;
acquérir des signaux de potentiels évoqués auditifs à l'aide des électrodes neurologiques (35) ; et
traiter les signaux de potentiels évoqués auditifs acquis à l'aide de l'unité de base portative (42).

13. Appareil selon la revendication 12, conçu en outre pour afficher les formes d'onde de signaux de potentiels évoqués auditifs et une mesure du rapport signal sur bruit associé aux signaux de potentiels évoqués.

14. Appareil selon la revendication 1, conçu en outre pour afficher les signaux électriques cérébraux acquis en temps réel.

15. Appareil selon la revendication 1, ledit processeur de signaux numériques (51) étant conçu pour calculer l'indice par les étapes de :

réalisation d'une conversion analogique-numérique des signaux ;
identification automatique et suppression des artefacts issus des signaux ; et
extraction de caractéristiques quantitatives et calcul des scores de transformée en Z.

**16.** Appareil selon la revendication 15, ledit calcul des scores de transformée en Z et la classification par discriminant étant effectués à l'aide d'une base de données démographiques stockée comprenant des données d'activité électrique cérébrale provenant d'une pluralité d'individus en présence ou en l'absence de différents types d'états neurologiques aigus.

**17.** Appareil selon la revendication 1, conçu en outre pour mesurer périodiquement l'impédance de chaque électrode.

**FIG. 1A**

**FIG. 1B**

| DISCRIMINANT CLASSIFICATION | 09:54 | 9/1/2008 |
|---|---|---|
| AVAIL. MEM 47 MIN. | AC ON | 74% |

TEST CONDUCTED WITH IMPEDANCE[s] > 10kOhm.
INTERPRET WITH CAUTION.

PROBABILITY OF NORMAL:
THIS PATIENT'S FRONTAL DISCRIMINANT SCORE LIES
OUTSIDE ($p<=0.025$) THE NORMAL LIMITS EXPECTED
FOR AN INDIVIDUAL OF THIS AGE.

0    3                                                      100%

THIS CLASSIFICATION IS A MULTIVARIATE STATISTICAL
SUMMARY OF A QUANTITATIVE EVALUATION OF BRAIN
ELECTRICAL ACTIVITY AND SERVES ONLY AS AN ADJUNCT
TO OTHER CLINICAL EVALUATIONS.

SELECT SESSION          DETAILED DATA

# FIG. 2A

DETAILED DATA          16:07          28.8.2008

AVAIL. MEM 47 MIN.      AC ON      ▮▮▮    74%

THE FEATURES MAKING THE LARGEST CONTRIBUTION TO THE "PROBABILITY OF NORMAL" STATEMENT ARE:

NORMED MONOPOLAR ABSOLUTE POWER COMBINED FOR ANTERIOR

NORMED MONOPOLAR COHERENCE COMBINED FOR ANTERIOR

NORMED BIPOLAR RELATIVE POWER BETA FOR HEAD

| ABSOLUTE POWER | RELATIVE POWER | MEAN FREQUENCY |
|---|---|---|
| COHERENCE | SYMMETRY | FRACTAL DIMENSION |
| BACK | HARMONICS STATISTICS | Z-SCORE NORMALIZED MAPS |

## FIG. 2B

EP 2 512 332 B1

FIG. 2C

18

FIG. 3

## REFERENCES CITED IN THE DESCRIPTION

**Patent documents cited in the description**

- US 20070032737 A
- US 20080208073 A
- US 20090263034 A
- US 20100191139 A